# EUROPÄISCHE PATENTSCHRIFT

(11) **EP 0 972 454 B1**
(45) Veröffentlichungstag und Bekanntmachung des Hinweises auf die Patenterteilung: **15.10.2003**
(21) Anmeldenummer: 99113420.6
(22) Anmeldetag: 12.07.1999
(51) Int. Cl.: A23L 1/272, A23L 1/275

(54) **Verwendung von Schwefel-organischen Verbindungen als Mittel zur bathochromen Verschiebung der UV/Vis-Absorptionsbande von Carotinoiden**
Use of sulfur containing organic compounds as agents for bathochromically shifting the UV/vis-absorption bands of carotenoids
Utilisation de composés organiques contenant du soufre comme agents pour déplacer bathochromiquement les bandes d'absorption dans l'uv et le visible des caroténoides

(30) Priorität: 16.07.1998 DE 19831865
(43) Veröffentlichungstag der Anmeldung: 19.01.2000
(73) Patentinhaber: BASF AKTIENGESELLSCHAFT, 67056 Ludwigshafen (DE)
(72) Erfinder: Auweter, Helmut, Dr., 67117 Limburgerhof (DE); Bohn, Heribert, 67319 Wattenheim (DE); Horn, Dieter, Dr., 69120 Heidelberg (DE); Krämer, Klaus, Dr., 76829 Landau (DE); Paust, Joachim, Dr., 67141 Neuhofen (DE); Weiss, Horst, Dr., 67141 Neuhofen (DE)

(56) Entgegenhaltungen:
- EP-A- 0 551 638
- GB-A- 921 306
- F. FEICHTMAYER ET AL: TETRAHEDRON, Bd. 25, Nr. 22, 1969, Seiten 5383-5408, XP002124303
- CHEMICAL ABSTRACTS, vol. 45, no. 1, 10. Januar 1951 (1951-01-10) Columbus, Ohio, US; abstract no. 372f, XP002124304 & DK 71 108 A (J.E. NYROP)

## Beschreibung

Die Erfindung betrifft die Verwendung von Schwefel-organischen Verbindungen in Komplexen mit Carotinoiden als Mittel zur bathochromen Verschiebung der Absorptionsbande von Carotinoiden im UV/Vis-Spektrum. Ferner betrifft die Erfindung Carotinoid-Formulierungen, die diese Komplexe enthalten, Verfahren zur Herstellung dieser Formulierungen sowie ihre Verwendung im Lebensmittel-, Kosmetik- und Pharmabereich.

Carotinoide bilden eine Gruppe von Farbpigmenten mit gelber bis roter Farbtonnuance, die in der Natur weitverbreitet vorkommen und vielen Nahrungsmitteln eine charakteristische Färbung verleihen. Als wichtigste Vertreter dieser Stoffklasse seien β-Carotin, β-Apo-8'-carotinal, Canthaxanthin, Asthaxanthin, Lycopin und Citranaxanthin genannt. Sowohl für die Lebensmittelindustrie als auch für die pharmazeutische Technologie stellen diese synthetisch herstellbaren Substanzen z.B. als Ersatz für künstliche Farbstoffe wichtige Farbkörper dar und sind zum Teil wegen ihrer Pro-Vitamin-A-Aktivität sowie darüberhinaus auch als Antioxidantien von großem Interesse.

Zum Färben von Lebensmitteln gewinnen natürliche oder naturidentische Lebensmittelfarbstoffe immer mehr an Bedeutung. Ein Hauptgrund für diesen Trend liegt zweifelsohne in der mangelnden Akzeptanz synthetischer Lebensmittelfarbstoffe bei den Verbrauchern.

Bei der Verwendung der o.g. Carotinoide als Farbstoff lassen sich je nach Vertreter dieser Stoffklasse bzw. je nach verwendeter Carotinoid-Formulierung unterschiedliche Farbtöne von hellgelb bis dunkelrot erzielen

Um mit Carotinoiden breitere Farbspektren abdecken zu können wird auf diesem Gebiet stets an der Entwicklung neuer Formulierungen gearbeitet.

So sind zur Verbesserung der Farbausbeuten verschiedene Verfahren beschrieben worden, die alle das Ziel haben, die Kristallitgröße der Wirkstoffe zu verkleinern und auf einen Teilchengrößenbereich von kleiner 10 µm zu bringen. Neben der Vermahlung von Carotinoiden, gemäß WO 91/06292 bzw. WO 94/19411, zählen dazu beispielsweise die bekannten Emulgier- und Mikronisierverfahren, u.a. beschrieben in DE-A-12 11 911, EP-A-0 410 236 sowie in EP-B-0 065 193.

In der EP-A-0 551 638 werden Emulsionen von β-Carotin beschrieben, die mit Ascorbylpalmitat als Emulgator stabilisiert sind, WO 94/06310 beschreibt die Verwendung von Carotinoid-Solubilisaten zur Getränkefärbung.

F.Feichtmayr et al: "Tetrahedron", Bd. 25, Nr. 22, 1969, Seiten 5383-5408, offenbart die Verwendung von CS₂ und Hexan als Mittel zur bathochromen Verschiebung der UV / Vis-Absorptionsbände von β-Carotin, Lycopin und anderen Carotinoiden. GB-A-921 306 beschreibt die Verwendung von 2-Naphthylthiol zum Schutz von β-Carotin vor oxidativem Abbau.

Aus "Carotenoids, Band 1B, Verlag Birkhäuser 1995, Basel, Hrsg. G. Britton, Seite 43" ist bekannt, daß die UV/Vis-Spektren von in Schwefelkohlenstoff gelösten Carotinoiden eine bathochrome Verschiebung von 30 bis 40 nm, bezogen auf das UV/Vis-Spektrum von Carotinoiden, gelöst in Kohlenwasserstoffen, zeigen. Die Carotinoide weisen demnach eine rötlichere Farbtonnuance auf, was wiederum für bestimmte Anwendungen, beispielsweise für die Getränkefärbung häufig gewünscht ist.

Aufgrund seiner stark toxischen Wirkung, u.a. auf das Nervensystem, steht eine Verwendung des anorganischen Schwefelkohlenstoffs z.B. als Lösungs- oder Dispergiermittel für Carotinoide im Lebensmittelbereich außer Frage.

Es war daher die Aufgabe, neue stabile Carotinoid-Formulierungen bereitzustellen, mit denen neue Farbtöne erzielt werden können, und die die oben genannten Nachteile des Standes der Technik nicht aufweisen.

Diese Aufgabe wurde erfindungsgemäß gelöst durch die Verwendung von Schwefel-organischen Verbindungen als Mittel zur bathochromen Verschiebung der Absorptionsmaxima von Carotinoiden im UV/Vis-Spektrum.

So wurde überraschenderweise gefunden, daß Carotinoide in Komplexen mit Schwefel-organischen Verbindungen eine Änderung ihrer Farbe hin zu rötlicheren Farbtönen, verbunden mit einer bathochromen Verschiebung ihrer Absorptionsmaxima zeigen.

Dieser Effekt war umso überraschender, da, im Gegensatz zu Schwefelkohlenstoff, die verwendeten Schwefel-organischen Verbindungen, keine Lösungsmittel darstellen und darüberhinaus andere Polaritäten als CS₂ besitzen.

Mit Komplexen sind hierbei im weitesten Sinne Stoffe gemeint, in denen durch Zusammenschluß von Carotinoiden und Schwefel-organischen Verbindungen intermolekulare Wechselwirkungen entstehen, bei denen die oben beschriebenen Verschiebungen der Absorptionsmaxima beobachtet werden. Dabei kann es sich u.a. um Addukte, Assoziate, Aggregate oder Einschlußverbindungen handeln.

Die durch die Schwefel-organischen Verbindungen verursachte bathochrome Verschiebung der Absorptionsmaxima der Carotinoide im UV/Vis-Spektrum liegt im Bereich von 1 bis 100 nm, bevorzugt im Bereich von 2 bis 60 nm, besonders bevorzugt im Bereich von 3 bis 50 nm. Als bathochrome Verschiebung ist hier die Änderung der Wellenlänge der Absorptionsmaxima der jeweiligen Carotinoide in den Komplexen in Relation zu den gemessenen Spektren der Carotinoide ohne Zusatz der erfindungsgemäß verwendeten Schwefel-organischen Verbindungen zu verstehen.

Bei den eingesetzten Schwefel-organischen Verbindungen kann es sich u.a. um die folgenden Schwefel-haltigen Naturstoffe oder deren Derivate handeln:
Aminosäuren wie Cystin, Cystein, N-Acetylcystein, S-Propylcystein, S-Allylcystein oder Methionin;
Bestandteile des Knoblauchs, z.B. Diallylthiosulfinat, S-Allylcysteinsulfoxid, Vinyldithiine, Ajoen;
Allithiamine wie Benfotiamin, Fursultiamin, Octotiamin oder Bentiamin;
Glutathion und dessen Ester, wie z.B. GSH-monomethylester, GSHdimethylester, GSH-monoethylester, GSH-diethylester.

Bevorzugte Schwefel-organische Verbindungen sind Liponsäure sowie besonders bevorzugt Liponsäurederivate. Bei der Liponsäure kann es sich dabei um racemische oder enantiomerenreine (R)- oder (S)-Liponsäure handeln. Als Liponsäurederivate seien u.a. folgende Verbindungen zu verstehen: Liponsäure-C₁-C₂₀-alkylester, Liponsäure-C₁-C₂₀-alkylamide, Dihydroliponsäure, Dihydroliponsäure-C₁-C₂₀-alkylester, Dihydroliponsäure-C₁-C₂₀-alkylamide, sowohl in racemischer als auch in optisch reiner Form.

Als Alkylreste der Ester bzw. Amide der Liponsäure bzw. Dihydroliponsäure seien verzweigte oder unverzweigte C₁-C₂₀-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-l-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als bevorzugte Alkylreste seien Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 2-Ethylhexyl genannt.

Die Carotinoide, die zusammen mit den erfindungsgemäß verwendeten Schwefel-organischen Verbindungen die eingangs genannten Komplexe bilden können, sind die bekannten, natürlichen oder synthetischen Vertreter dieser Klasse, wie z.B. β-Carotin, Lycopin, Bixin, Zeaxanthin, Cryptoxanthin, Citranaxanthin, Lutein, Canthaxanthin, β-Apo-4-carotinal, β-Apo-8-carotinal, β-Apo-8-carotinsäureester, Astaxanthin, einzeln oder als Mischung. Besonders bevorzugt werden die technisch gut zugänglichen Vertreter wie β-Carotin, Canthaxanthin, β-Apo-8-carotinal, Astaxanthin oder Lycopin, insbesondere β-Carotin, Astaxanthin und/oder Lycopin verwendet.

Die Mengenverhältnisse (Gewichtsverhältnisse) von Carotinoiden zu den Schwefel-organischen Verbindungen in den Komplexen liegen im Bereich von 1:0,001 bis 1:1000, bevorzugt im Bereich von 1:0,01 bis 1:100, besonders bevorzugt im Bereich von 1:0,02 bis 1:50, ganz besonders bevorzugt im Bereich von 1:1 bis 1:10.

Die Herstellung der Komplexe kann im einfachsten Fall durch Mischen eines oder mehrerer Carotinoide mit mindestens einer Schwefel-organischen Verbindung, gegebenenfalls in einem Lösungsmittel, beispielsweise in chlorierten Kohlenwasserstoffen, wie Methylenchlorid oder Chloroform, oder in einem Alkohol, beispielsweise Isopropanol erfolgen. Zur Erhöhung der Löslichkeit kann es gegebenenfalls von Vorteil sein, das Gemisch kurzzeitig zu erwärmen.

Gegenstand der Erfindung sind ebenfalls Carotinoid-Formulierungen, enthaltend
a) mindestens einen Komplex aus mindestens einem Carotinoid und mindestens einer Schwefel-organischen Verbindung sowie
b) mindestens einen weiteren Hilfs- oder Zusatzstoff.

Ebenso wie bei den oben beschriebenen Komplexen sind bei den daraus hergestellten Carotinoid-Formulierungen bathochrome Verschiebungen der Absorptionsmaxima im UV/Vis-Spektrum der Carotinoide gefunden worden.

Diese liegen ebenfalls im Bereich von 1 bis 100 nm, bevorzugt im Bereich von 2 bis 60 nm, besonders bevorzugt im Bereich von 3 bis 50 nm.

Die in den Carotinoid-Formulierungen in Form der eingangs erläuterten Komplexe vorliegenden Schwefel-organischen Verbindungen sind u.a. die bereits genannten Schwefel-haltigen Naturstoffe.

Bevorzugte Carotinoid-Formulierungen sind solche, die Liponsäure oder die bereits näher beschriebenen Liponsäurederivate enthalten.

Der Gehalt an Carotinoiden in den erfindungsgemäßen Formulierungen liegt im allgemeinen zwischen 0,01 und 25 Gew.-%, bevorzugt zwischen 1 und 20 Gew.-%, besonders bevorzugt zwischen 5 und 15 Gew.-%, bezogen auf die Gesamtmenge der Formulierung.

Der Gehalt an Schwefel-organischen Verbindungen in den erfindungsgemäßen Formulierungen liegt im allgemeinen zwischen 0,1 und 40 Gew.-%, bevorzugt zwischen 1 und 30 Gew.-%, besonders bevorzugt zwischen 2 und 25 Gew.-%, bezogen auf die Gesamtmenge der Formulierung.

Mit dem Begriff "Carotinoid-Formulierungen" sind im Rahmen dieser Erfindung sowohl Lösungen, Solubilisate und Dispersionen, wie Emulsionen oder Suspensionen als auch daraus hergestellte Trokkenpulver von Carotinoiden gemeint.

Von der Art der Carotinoid-Formulierung hängt es beispielsweise ab, welche Schwefel-organische Verbindung als Mittel zur bathochromen Verschiebung der Absorptionsmaxima von Carotinoiden besonders geeignet sind. Von Vorteil kann es sein, wenn die Lipophilie der Schwefel-organischen Verbindung der Carotinoid-Formulierung angepaßt ist. So sind in ölhaltigen Carotinoid-Formulierungen beispielsweise lipophile Liponsäureester oder Amide von Vorteil. In alkoholischen Lösungen von Carotinoiden dagegen lassen sich beispielsweise auch die polareren Schwefel-haltigen Aminosäuren sowie Liponsäure oder Dihydroliponsäure für die o.g. Anwendung einsetzen.

Neben den als Komponente a) bezeichneten Komplex(en) enthalten die Formulierungen mindestens einen weiteren Hils- oder Zusatzstoff, wie z.B. Schutzkolloide, Öle, Weichmacher, Antioxidantien und/oder Emulgatoren.

Als Schutzkolloide werden beispielsweise Gelatine, Fischgelatine, Stärke, Dextrin, Pflanzenproteine, Pektin, Gummi-Arabikum, Kasein, Kaseinat oder Mischungen davon verwendet. Es können aber auch Polyvinylalkohol, Polyvinylpyrrolidon, Methylcellulose, Carboxymethylcellulose, Hydroxypropylcellulose und Alginate eingesetzt werden. Bezüglich näherer Einzelheiten wird auf R.A. Morton, Fat Soluble Vitamins, Intern. Encyclopedia of Food and Nutrition, Bd.9, Pergamon Press 1970, S. 128-131, verwiesen. Zur Erhöhung der mechanischen Stabilität beispielsweise des Trockenpulvers ist es zweckmäßig, dem Kolloid einen Weichmacher zuzusetzen, wie Zucker oder Zuckeralkohole, z.B. Saccharose, Glucose, Lactose, Invertzucker, Sorbit, Mannit oder Glycerin.

Das Verhältnis Schutzkolloid und Weichmacher zu Carotinoidlösung wird im allgemeinen so gewählt, daß als Endprodukt eine Formulierung erhalten wird, die neben den oben genannten Carotinoiden und Schwefel-organischen Verbindungen 10 bis 50 Gew.-% eines Schutzkolloids, 20 bis 70 Gew.-% eines Weichmachers, alle Prozentangaben bezogen auf die Trockenmasse der Formulierung, sowie gegebenenfalls untergeordnete Mengen eines Stabilisators enthält.

Zur Erhöhung der Stabilität des Wirkstoffes gegen oxidativen Abbau ist es vorteilhaft, Stabilisatoren wie α-Tocopherol, t-Butylhydroxy-toluol, t-Butylhydroxyanisol, Ascorbinsäure oder Ethoxyquine zuzusetzen.

Als Emulgatoren können beispielsweise Ascorbylpalmitat, Polyglycerin-Fettsäureester, Sorbitan-Fettsäureester, Propylenglycol-Fettsäureester oder Lecithin in einer Konzentration von 0 bis 200 Gew.%, vorzugsweise 10 bis 150 Gew.%, besonders bevorzugt 20 bis 80 Gew.%, bezogen auf das/die Carotinoid(e), verwendet werden.

Unter Umständen kann es auch vorteilhaft sein, im Falle einer Dispersion, insbesondere im Falle einer Emulsion, zusätzlich in der organischen Phase ein physiologisch zugelassenes Öl wie beispielsweise Sesamöl, Maiskeimöl, Baumwollsaatöl, Sojabohnenöl oder Erdnußöl, Ester mittelkettiger pflanzlicher Fettsäuren sowie außerdem Fischöle wie beispielsweise Makrelen-, Sprotten- oder Lachsöl in einer Konzentration von 0 bis 500 Gew.%, vorzugsweise 10 bis 300 Gew.%, besonders bevorzugt 20 bis 100 Gew.%, bezogen auf das/die Carotinoid(e), zu lösen, das dann gemeinsam mit den Wirkstoffen und den genannten Zusatzstoffen beim Mischen mit der wässrigen Phase extrem feinteilig ausgefällt wird.

Neben den Komponenten a) und b) können die erfindungsgemäßen Carotinoid-Formulierungen mindestens einen weiteren Wirkstoff in Konzentrationen von 0,01 bis 40 Gew.-%, bevorzugt 0,1 bis 30 Gew.-%, besonders bevorzugt in Konzentrationen von 0,5 bis 20 Gew.-% enthalten.

Dabei kann es sich u.a um folgende Wirkstoffe handeln:

Vitamine, z.B. Vitamin A, Vitamin A-Acetat, Vitamin A-Palmitat, Riboflavin, Vitamin B₁₂, Ascorbinsäure, Nicotinsäure, Nicotinsäureamid, Pyridoxin Hydrochlorid, Vitamin D₃, Tocopherol, Tocopherolacetat, Tocotrienol, Vitamin K, Thiamin, Calcium-Pantothenat, Biotin, Folsäure, Folsäurederivate wie Tetrahydrofolsäure, 5-Methyl-tetrahydrofolsäure, 10-Formyl-tetrahydrofolsäure oder 5-Formyl-tetrahydrofolsäure.

Verbindungen mit Vitamin- oder Coenzymcharakter, z.B. Cholinchlorid, Carnitin, Taurin, Kreatin, Ubichinone, S-Methylmethionin, S-Adenosylmethionin.

Mehrfach ungesättigte Fettsäuren, z.B. Linolsäure, Linolensäure, Arachidonsäure, Eicosapentaensäure, Docosahexaensäure.

Die Herstellung der Carotinoid-Formulierungen erfolgt in an sich bekannter Weise. So lassen sich beispielsweise Solubilisate bzw. Emulsionen gemäß US 4,435,427 bzw. EP-A-0 551 638 herstellen. Die Herstellung von Carotinoid-Dispersionen und deren Überführung in ein Trockenpulver ist u.a. beschrieben in EP-A-0 065 193 und EP-A-0 410 236.

Die zur Fällung verwendeten Alkohole sind z.B. Methanol, Ethanol, n-Propanol, iso-Propanol, n-Butanol, tert.-Butanol, bevorzugt Methanol oder Ethanol. Als Ketone eignen sich Aceton, Methylethylketon, Diethylketon, bevorzugt Aceton. Als cyclischer Ether wird bevorzugt Tetrahydrofuran eingesetzt.

Ein bevorzugtes Herstellverfahren besteht darin, daß ein Carotinoid zusammen mit einer Schwefel-organischen Verbindung und weiteren, bereits oben genannten Hilfs- oder Zusatzstoffen in einem mit Wasser mischbaren Lösungsmittel, beispielsweise Methanol, Ethanol, n-Propanol, iso-Propanol, n-Butanol, tert.-Butanol, Aceton, Methylethylketon, Diethylketon oder Tetrahydrofuran gelöst wird, wobei Lösedauer, Lösetemperatur, Druck und Energieeintrag durch Rühren oder statisches Vermischen den Löseeigenschaften des Carotinoids und der Schwefel-organischen Verbindung angepaßt sind. Diese Lösung kann dann mit einer wäßrigen Phase turbulent vermischt werden, wobei das Carotinoid und die Schwefel-organische Verbindung gemeinsam in Form von Nanopartikeln gefällt werden. Die wäßrige Phase enthält dabei in der Regel ein Schutzkolloid und kann außerdem weitere Hilfs- und Zusatzstoffe enthalten. Die so gewonnene nanopartikuläre Dispersion kann aufkonzentriert und nach bekannten Verfahren, beispielsweise mittels Sprühtrocknung in ein Trockenpulver überführt werden.

Eine weitere Möglichkeit zur Herstellung der erfindungsgemäßen Trockenpulver besteht darin, daß ein Carotinoid zusammen mit einer Schwefel-organischen Verbindung und gegebenenfalls weiteren Hilfs- bzw. Zusatzstoffen in einem mit Wasser nicht mischbaren Lösungsmittel, beispielsweise Hexan, Cyclohexan, Toluol, Methylenchlorid, Chloroform oder Ethylacetat gelöst wird. Diese Lösung kann dann in eine wäßrige Phase emulgiert werden. Die wäßrige Phase enthält dabei in der Regel einen Emulgator und kann außerdem weitere Hilfs- und Zusatzstoffe enthalten. Anschließend können Lösungsmittel und Wasser abdestilliert werden, wobei das Carotinoid und die Schwefel-organische Verbindung gemeinsam in Form von Nanopartikeln gefällt werden. Die so gewonnene konzentrierte Dispersion läßt sich anschließend ebenfalls in ein Trockenpulver überführen.

Die erfindungsgemäßen Carotinoid-Formulierungen eignen sich u.a. als Zusatzstoff für die Herstellung von Präparaten zur Nahrungsergänzung im Human- und Tierbereich sowie für die Herstellung pharmazeutischer und kosmetischer Präparate.

Aufgrund ihrer guten Kaltwasserdispergierbarkeit eignen sich die Trockenpulver hervorragend als Lebensmittelfarbstoffe, beispielsweise für Erfrischungsgetränke. Sie können auch anderen Lebensmitteln, beispielsweise Milchprodukten wie Joghurt, Milchmixgetränken oder Milchspeiseeis sowie Puddingpulvern oder Backmischungen zugegeben werden.

In den folgenden Beispielen wird die erfindungsgemäße Verwendung der Schwefel-organischen Verbindungen als Mittel zur bathochromen Verschiebung der UV/Vis-Absorptionsbande von Carotinoiden näher erläutert.

### Beispiel 1:

25 mg Astaxanthin wurden mit 10 g (R,S)-Dihydro-Liponsäure gemischt und bei 40°C 60 Minuten intensiv gerührt. Nach Abkühlen auf Raumtemperatur wurde die leicht trübe Mischung über ein 0,2 µm Filter abfiltriert und zur Messung des UV/Vis - Absorptionsspektrums in eine 1 cm Küvette überführt.

Das Absorptionsspektrum, gemessen im Bereich zwischen 350 und 650 nm, zeigte ein Maximum bei 500 nm. Im Vergleich zum Referenzspektrum von Astaxanthin, gelöst in Cyclohexan (λₘₐₓ = 474nm) lag die bathochrome Verschiebung bei 26 nm.

### Beispiel 2

25 g Astaxanthin, 75 g (R)-Alpha-Liponsäuremethylester, 2 g Ascorbylpalmitat und 7,7 g Ethoxiquin wurden in einem Lösungsmittelgemisch von 450 g Isopropanol und 400 g Tetrahydrofuran als Suspension angesetzt und mit einem Ultra-Turrax bei Raumtemperatur für 30 Minuten kräftig gerührt. Die wäßrige Phase wurde wie folgt angesetzt: 88 g Gelatine B 200 Bloom, 42 g Gelita Sol P, 146 g Saccharose und 5,01 g Konservierungsmittel wurden in 14 l destilliertem Wasser gelöst, wobei anschließend der pH-Wert der Lösung mittels 52 ml 1 M NaOH von pH 5 auf pH 8,5 angehoben wurde. Bei einer Temperatur von 139° C und einem Druck von 60 bar wurde die Suspension in eine molekulardisperse Lösung überführt und durch turbulentes Vermischen mit der wäßrigen Phase gefällt.

Durch Abdestillieren der Lösungsmittel und des Wassers und durch anschließende Doppelemulgierung wurde ein Trockenpulver hergestellt, das einen Astaxanthingehalt von 7 Gew.-% aufweist bei einem Liponsäuremethylestergehalt von 21 Gew.-%. Das in Wasser redispergierte Trockenpulver wies eine Teilchengröße von 380 nm und einen E1/1-Wert von 89 auf. Die bathochrome Verschiebung im Vergleich zu einer Referenzformulierung ohne Liponsäuremethylester betrug 4 nm.

## Patentansprüche

1. Verwendung von Schwefel-organischen Verbindungen, ausgewählt aus der Gruppe, bestehend aus Cystin, Cystein, N-Acetylcystein, S-Propylcystein, S-Allylcystein, Methionin, Diallylthiosulfinat, S-Allylcysteinsulfoxid, Vinyldithiine, Ajoen, Benfotiamin, Fursultiamin, Octotiamin, Bentiamin, Glutathion und dessen Ester, Liponsäure und Liponsäurederivate als Mittel zur bathochromen Verschiebung der UV-Absorptionsbande von Carotinoiden.

2. Verwendung nach Anspruch 1, **dadurch gekennzeichnet, daß** die Carotinoide im UV/Vis-Spektrum eine bathochrome Verschiebung des Absorptionsmaximums von 1 bis 100 nm zeigen.

3. Verwendung nach den Ansprüchen 1 und 2, **dadurch gekennzeichnet, daß** es sich bei den Schwefel-organischen Verbindungen um Liponsäure und/oder Liponsäurederivate handelt.

4. Verwendung nach Anspruch 3, **dadurch gekennzeichnet, daß** es sich bei den Schwefel-organischen Verbindungen um Liponsäurederivate, ausgewählt aus der Gruppe, bestehend aus Dihydroliponsäure, Liponsäure-C₁-C₂₀-alkylester und Liponsäure-C₁-C₂₀-alkylamide handelt.

5. Komplexe aus mindesten einem Carotinoid und mindestens einer Schwefel-organischen Verbindung, ausgewählt aus der Gruppe, bestehend aus Cystin, Cystein, N-Acetylcystein, S-Propylcystein, S-Allylcystein, Methionin, Diallylthiosulfinat, S-Allylcysteinsulfoxid, Vinyldithiine, Ajoen, Benfotiamin, Fursultiamin, Octotiamin, Bentiamin, Glutathion und dessen Ester, Liponsäure und Liponsäurederivate.

6. Komplexe nach Anspruch 5, **dadurch gekennzeichnet, daß** sie im UV/Vis-Spektrum eine bathochrome Verschiebung des UV-Absorptionsmaximums der Carotinoide zeigen.

7. Komplexe nach den Ansprüchen 5 und 6, **dadurch gekennzeichnet, daß** sie als Schwefel-organische Verbindung Liponsäure und/- oder mindestens ein Liponsäurederivat enthalten.

8. Komplexe nach Anspruch 7, **dadurch gekennzeichnet, daß** sie als Schwefel-organische Verbindung Liponsäurederivate, ausgewählt aus der Gruppe, bestehend aus Dihydroliponsäure, Liponsäure-C₁-C₂₀-alkylester und Liponsäure-C₁-C₂₀-alkylamide enthalten.

9. Carotinoid-Formulierungen, enthaltend
a) mindestens einen Komplex, definiert gemäß Anspruch 5 sowie
b) mindestens einen weiteren Hilfs- oder Zusatzstoff.

10. Carotinoid-Formulierungen nach Anspruch 9, mit einem Carotinoid Gehalt von 0,01 bis 25 Gew.-% und mit einem Gehalt an Schwefel-organischen Verbindungen von 0,1 bis 40 Gew.-%.

11. Carotinoid-Formulierungen nach den Ansprüchen 9 und 10, die als Schwefel-organische Verbindung Liponsäure und/oder mindestens ein Liponsäurederivat enthalten.

12. Carotinoid-Formulierungen nach Anspruch 9, enthaltend zusätzlich 0,01 bis 40 Gew.-% eines oder mehrerer weiterer Wirkstoffe.

13. Carotinoid-Formulierungen nach Anspruch 9, **dadurch gekennzeichnet, daß** die darin enthaltenen Carotinoide im UV/Vis-Spektrum eine bathochrome Verschiebung des Absorptionsmaximums von 1 bis 100 nm zeigen.

14. Carotinoid-Formulierungen nach Anspruch 9, **dadurch gekennzeichnet, daß** es sich dabei um Lösungen, Solubilisate, Dispersionen oder Trockenpulver handelt.

15. Verfahren zur Herstellung von Carotinoid-Formulierungen, definiert gemäß Anspruch 9, **dadurch gekennzeichnet, daß** man mindestens ein Carotinoid und mindestens eine Schwefel-organische Verbindung in Wasser oder einer wäßrigen Schutzkolloidlösung dispergiert und diese Dispersion gegebenenfalls trocknet.

16. Verfahren nach Anspruch 15, **dadurch gekennzeichnet, daß** man das Carotinoid zusammen mit der Schwefel-organischen Verbindung vor dem Dispergierschritt in einem wassermischbaren organischen Lösungsmittel löst.

17. Verwendung von Carotinoid-Formulierungen, definiert gemäß Anspruch 9, als Zusatzstoff für die Herstellung von Präparaten zur Nahrungsergänzung im Human- und Tierbereich sowie kosmetischer und pharmazeutischer Präparate.

18. Verwendung von Carotinoid-Formulierungen, definiert gemäß Anspruch 9 als Lebensmittelfarbstoff.

19. Nahrungsergänzungsmittel, Lebensmittel, Tierfuttermittel sowie kosmetische und pharmazeutische Zubereitungen, enthaltend Carotinoidformulierungen definiert gemäß Anspruch 9.

## Claims

1. The use of organosulfur compounds selected from the group consisting of cystine, cystein, N-acetylcystein, S-propylcystein, S-allylcystein, methionine, diallyl thiosulfinate, S-allylcystein sulfoxide, vinyldithiine, ajoene, benfotiamine, fursultiamine, octotiamine, bentiamine, glutathione and its esters, lipoic acid and lipoic acid derivatives as agents for inducing bathochromic shifts in the UV absorption bands of carotenoids.

2. The use as claimed in claim 1, wherein the carotenoids display a bathochromic shift of the absorption maximum in the UV/vis spectrum of from 1 to 100 nm.

3. The use as claimed in claim 1 or 2, wherein the organosulfur compounds are lipoic acid and/or lipoic acid derivatives.

4. The use as claimed in claim 3, wherein the organosulfur compounds are lipoic acid derivatives selected from the group consisting of dihydrolipoic acid, C₁-C₂₀-alkyl lipoates and C₁-C₂₀-alkylamides of lipoic acid.

5. A complex of at least one carotenoid and at least one organosulfur compound selected from the group consisting of cystine, cystein, N-acetylcystein, S-propylcystein, S-allylcystein, methionine, diallyl thiosulfinate, S-allylcystein sulfoxide, vinyldithiine, ajoene, benfotiamine, fursultiamine, octotiamine, bentiamine, glutathione and its esters, lipoic acid and lipoic acid derivatives.

6. The complex as claimed in claim 5 which displays a bathochromic shift of the UV absorption maximum in the UV/vis spectrum of the carotenoids.

7. The complex as claimed in claim 5 or 6 which contains lipoic acid and/or at least one lipoic acid derivative as organosulfur compound.

8. The complex as claimed in claim 7 which contains lipoic acid derivatives selected from the group consisting of dihydrolipoic acid, C₁-C₂₀-alkyl lipoates and C₁-C₂₀-alkylamides of lipoic acid as organosulfur compound.

9. A carotenoid formulation comprising
a) at least one complex as defined in claim 5 and
b) at least one further auxiliary or additive.

10. The carotenoid formulation as claimed in claim 9 having a carotenoid content of from 0.01 to 25% by weight and a content of organosulfur compounds of from 0.1 to 40% by weight.

11. The carotenoid formulation as claimed in claim 9 or 10 which contains lipoic acid and/or at least one lipoic acid derivative as organosulfur compound.

12. The carotenoid formulation as claimed in claim 9 which further comprises from 0.01 to 40% by weight of one or more further active compounds.

13. The carotenoid formulation as claimed in claim 9 in which the carotenoids present display a bathochromic shift of the absorption maximum in the UV/vis spectrum of from 1 to 100 nm.

14. The carotenoid formulation as claimed in claim 9 which is a solution, a solubilized material, a dispersion or a dry powder.

15. A process for producing carotenoid formulations as defined in claim 9, which comprises dispersing at least one carotenoid and at least one organosulfur compound in water or an aqueous protective colloid solution and, if desired, drying this dispersion.

16. The process as claimed in claim 15, wherein the carotenoid together with the organosulfur compound are dissolved in a water-miscible organic solvent prior to the dispersion step.

17. The use of a carotenoid formulation as defined in claim 9 as additive for the production of nutritional supplement preparations in the human and animal sectors and also cosmetic and pharmaceutical preparations.

18. The use of a carotenoid formulation as defined in claim 9 as food dye.

19. A nutritional supplement, a food, an animal feed or a cosmetic or pharmaceutical preparation comprising a carotenoid formulation as defined in claim 9.

## Revendications

1. Utilisation de composés organiques contenant du soufre, choisis dans le groupe consistant en cystine, cystéine, N-acétylcystéine, S-propylcystéine, S-allylcystéine, méthionine, diallylthiosulfinate, sulfoxyde de S-allylcystéine, vinyldithiine, ajoène, benfotiamine, fursultiamine, octotiamine, bentiamine, glutathion et ses esters, acide lipoïque et dérivés d'acide lipoïque, comme agents pour le déplacement bathochromique des bandes d'absorption dans l'UV des caroténoïdes.

2. Utilisation selon la revendication 1, **caractérisée par le fait que** les caroténoïdes présentent dans le spectre UV/visible un déplacement bathochrome du maximum d'absorption de 1 à 100 nm.

3. Utilisation selon les revendications 1 et 2, **caractérisée par le fait qu'**il s'agit, pour les composés organiques contenant du soufre, d'acide lipoïque et/ou de dérivés d'acide lipoïque.

4. Utilisation selon la revendication 3, **caractérisée par le fait qu'**il s'agit, pour les composés organiques contenant du soufre, de dérivés d'acide lipoïque choisis dans le groupe consistant en acide dihydrolipoïque, ester d'alkyle en C₁-C₂₀ d'acide lipoïque et amide d'alkyle en C₁-C₂₀ d'acide lipoïque.

5. Complexes d'au moins un caroténoïde et d'au moins un composé organique contenant du soufre, choisi dans le groupe consistant en cystine, cystéine, N-acétylcystéine, S-propylcystéine, S-allylcystéine, diallylthiosulfinate, S-allylcystéinesulfoxyde, vinyldithiine, ajoène, benfotiamine, fursultiamine, octotiamine, bentiamine, glutathion et ses esters, acide lipoïque et dérivés d'acide lipoïque.

6. Complexes selon la revendication 5, **caractérisés par le fait qu'**ils présentent dans le spectre UV/visible un déplacement bathochrome du maximum d'absorption dans l'UV des caroténoïdes.

7. Complexes selon les revendications 5 et 6, **caractérisés par le fait qu'**ils contiennent comme composé organique contenant du soufre de l'acide lipoïque et/ou au moins un dérivé d'acide lipoïque.

8. Complexes selon la revendication 7, **caractérisé par le fait qu'**ils contiennent comme composé organique contenant du soufre des dérivés d'acide lipoïque choisis dans le groupe consistant en acide dihydrolipoïque, ester d'alkyle en C₁-C₂₀ d'acide lipoïque et alkylamide en C₁-C₂₀ d'acide lipoïque.

9. Formulations de caroténoïdes, contenant
a) au moins un complexe défini selon la revendication 5, ainsi que
b) au moins un autre adjuvant ou additif.

10. Formulations de caroténoïdes selon la revendication 9, ayant une teneur en caroténoïde de 0,01 à 25 % en poids et ayant une teneur en composés organiques contenant du soufre de 0,1 à 40 % en poids.

11. Formulations de caroténoïdes selon les revendications 9 et 10, qui contiennent comme composé organique contenant du soufre de l'acide lipoïque et/ou au moins un dérivé d'acide lipoïque.

12. Formulations de caroténoïdes selon la revendication 9, contenant en outre 0,01 à 40 % en poids d'une ou plusieurs autres substances actives.

13. Formulations de caroténoïdes selon la revendication 9, **caractérisées par le fait que** les caroténoïdes qui y sont contenus présentent dans le spectre UV/visible un déplacement bathochrome du maximum d'absorption de 1 à 100 nm.

14. Formulations de caroténoïdes selon la revendication 9, **caractérisées par** e fait qu'il s'agit alors de solutions, de produits de solubilisation, de dispersions ou de poudres sèches.

15. Procédé pour la préparation de formulations de caroténoïdes, définis selon la revendication 9, **caractérisé par le fait qu'**on disperse au moins un caroténoïde et au moins un composé organique contenant du soufre dans l'eau ou une solution aqueuse de colloïde protecteur, et on sèche éventuellement cette dispersion.

16. Procédé selon la revendication 15, **caractérisé par le fait qu'**on dissout le caroténoïde conjointement avec le composé organique contenant du soufre avant l'étape de dispersion, dans un solvant organique miscible avec l'eau.

17. Utilisation de formulations de caroténoïdes, définies selon la revendication 9, comme additif pour l'élaboration de préparations pour complément alimentaire dans le domaine humain et animal, ainsi que de préparations cosmétiques et pharmaceutiques.

18. Utilisation de formulations de caroténoïdes, définies selon la revendication 9, comme colorants pour aliments.

19. Produits de complément alimentaire, aliments, aliments pour animaux, ainsi que préparations cosmétiques et pharmaceutiques, contenant des formulations de caroténoïdes définies selon la revendication 9.
